# EUROPEAN PATENT SPECIFICATION

(11) **EP 2 192 885 B1**
(45) Date of publication and mention of the grant of the patent: **20.02.2013**
(21) Application number: 08799020.6
(22) Date of filing: 29.08.2008
(51) Int. Cl.: A61J 15/00, A61M 25/01, A61M 25/00, B29C 47/00

(54) **Guided catheter with removable magnetic guide**
Geführter Katheter mit entfernbarer magnetischer Führung
Cathéter guidé comportant un guide magnétique amovible

(30) Priority: 30.08.2007 US 969020 P; 18.06.2008 US 73552
(43) Date of publication of application: 09.06.2010
(73) Proprietor: Syncro Medical Innovations, Inc., Youngstown, OH 44503 (US)
(72) Inventor: WAKEFORD, Gary, Canfield, OH 44406 (US); FRANKHOUSER, Paul, State College, PA 16801 (US); GABRIEL, Sabry, Lizella, GA 31052 (US); WINKLER, Josef, Wayland, MA 01778 (US); SANDOSKI, Aaron, Lincoln, MA 01773 (US)
(74) Representative: Jordan, Volker Otto Wilhelm
(86) International application number: PCT/US2008/074893
(87) International publication number: WO 2009/029869

(56) References cited:
- WO-A-03/041785
- WO-A-2007/025046
- DE-A1-102004 058 008
- US-A- 4 244 362
- US-A- 4 296 376
- US-A- 5 085 216
- US-A- 5 257 636
- US-A- 5 353 807
- US-A- 5 431 640
- US-A- 5 728 079
- US-A- 5 843 153
- US-A- 6 164 277
- US-A1- 2005 021 063
- US-A1- 2005 154 259
- US-A1- 2007 016 131
- US-B1- 7 066 924

## Description

### FIELD OF THE INVENTION

The present invention relates to medical catheters, particularly for use as feeding tubes.

### BACKGROUND OF THE INVENTION

U.S. Patent No. 5,431,640 to Gabriel discloses a method and apparatus for intubation of a patient using a magnetic field produced between an external magnet and a magnet at the distal end of a catheter to maneuver the catheter to the distal duodenum of a patient. An operator maneuvering an external magnet cannot see through the abdominal wall to decide whether the distal end portion of the catheter is continuously captured by the magnetic field of the external magnet during the advancement of the catheter. Thus, use of this particular device required an additional procedure, such as X-ray monitoring or aspiration of fluid from the distal end of the catheter and measurement of the pH of the aspirated fluid, to determine whether the distal end of the catheter had properly advanced into the patient's duodenum.

U.S. Patent No. 6,126,647 to Posey, et al., discloses a catheter guided by an external magnet, which contains a sensor that indicates whether the distal end of the catheter is being properly advanced into the patient's duodenum. The catheter contains a magnet that is permanently affixed in the distal portion of the catheter.

Although each of the above-described catheters provide improvements over other feeding tubes that are available, none of the feeding tubes is designed to be used with diagnostic imaging techniques, such as magnetic resonance imaging (MRI). Since the magnet is permanently affixed in the distal portion of the catheter, it interferes with MRI techniques. Although the feeding tubes are designed to deliver food and nutrients to a patient, they often are clogged with food particles, and then require removal for cleaning or replacement.

There is a need for improved, more versatile catheters for use as feeding tubes.

Therefore, it is an object of the invention to provide improved catheters for use as feeding tubes.

WO 2007/025046 A discloses a stylet for insertion into a patient. The stylet includes at its tip end a magnet that is used to provide an indication of the position of the stylet tip inside the patient.

### SUMMARY OF THE INVENTION

According to the invention there is provided a feeding tube apparatus as defined in Claim 1. Optional features are set out in the dependent claims.

A feeding tube apparatus, a kit containing the feed tube apparatus and method for intubating a patient to deliver the feeding tube apparatus to the desired location for delivering nutrients and/or medication are described herein. The feeding tube apparatus contains at least a catheter and a removable stylet, where the removable stylet contains one or more magnetic materials. In one embodiment, the stylet contains more than one magnetic material in the form of a magnet stack. The feeding tube apparatus is used in combination with a suitable external magnet which a medical practitioner can use to guide the feeding tube apparatus through the intestinal tract. Optionally, the feeding tube apparatus contains additional components, such as a spring wire guide. The feeding tube apparatus is inserted into the patient's body and the external magnet is applied to the patient's body within the minimum distance required to create a magnetic field between the external magnet and the magnetic material(s) located at the distal end of the stylet that is sufficiently strong to allow the external magnet to guide the catheter and stylet through the intestinal tract, and into the distal duodenum of the small intestine. Once the catheter is placed in the desired location, the stylet is removed, thereby removing the magnetic materials from, the feeding tube apparatus. This allows the catheter to remain in place while the patient undergoes diagnostic testing, such as magnetic resonance imaging.

### BRIEF DESCRIPTION OF THE DRAWINGS

Figure 1 is a schematic of the feeding tube apparatus.

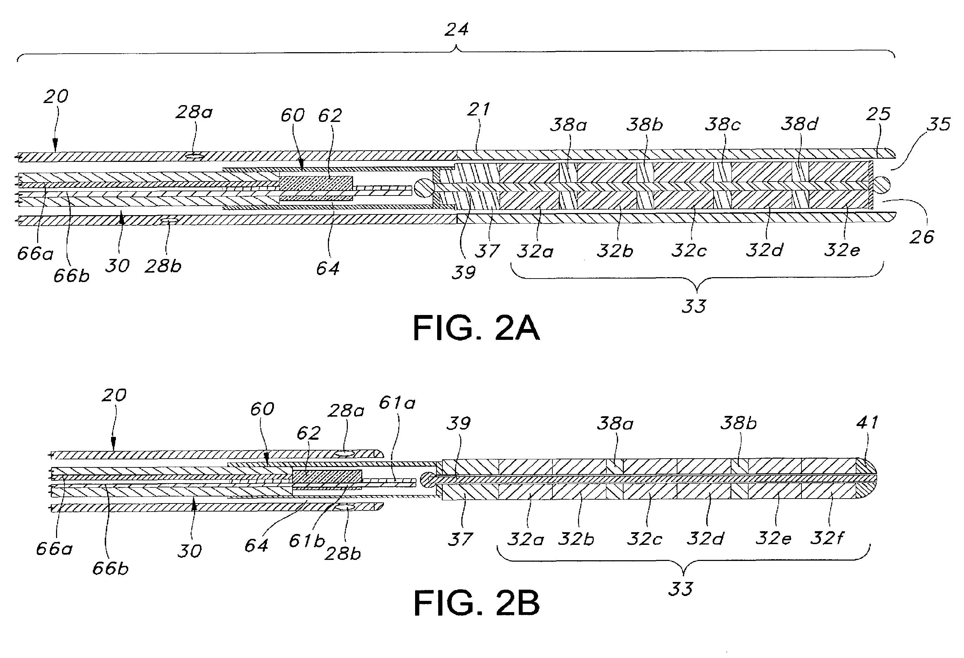

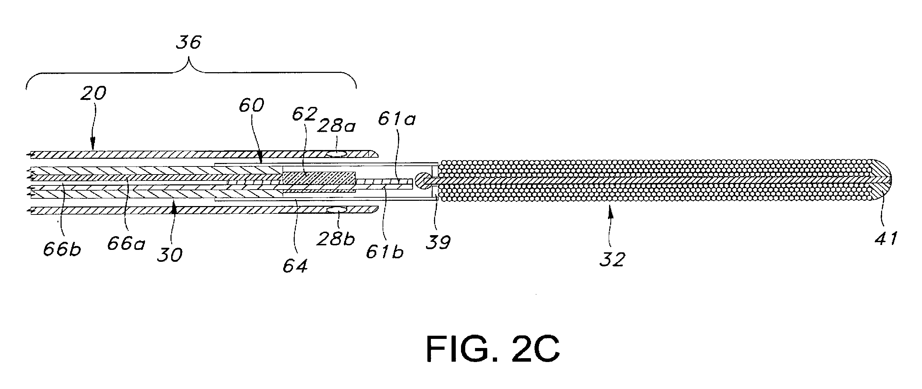

Figures 2A-C illustrate cross-sections of the distal tip of the feeding tube apparatus for three different embodiments of the apparatus. In Figure 2A the apparatus contains a magnet stack with spacers between each magnet in the magnet stack. In Figure 2B the magnetic stack is extended beyond the distal tip of the catheter, and the apparatus contains a magnet stack with three groups containing two magnetic materials, preferably magnets, where each group is separated from the next one by a spacer. In Figure 2C the magnetic material extended beyond the distal tip of the catheter, and the apparatus contains a continuous, flexible magnetic material in the form of a wound coil or stranded cable.
Figure 3 illustrates a cross-sectional view of a catheter formed of a reinforced material.
Figures 4A and 4B illustrate isometric views for the feeding tube hub (Fig. 4A) and stylet hub (Fig. 4B).
Figures 5A-C illustrate three representative configurations for the external magnet.
Figures 6A-C illustrate embodiments of the feeding tube apparatus in which the stylet contains a spring wire guide at its distal tip. In Figure 6A, the spring wire guide extends beyond the distal tip of the catheter. In Figure 6B, the spring wire guide is inside the catheter and extends until the distal tip of the catheter. In Figure 6C, the stylet is extended so that both the magnet stack and the spring wire guide extend beyond the distal tip of the catheter.
Figure 7 illustrates a cross-section of the reed switch assembly.
Figure 8 is an illustration showing the path of the catheter within anatomical quadrants during passage through the stomach to the distal duodenum of the small intestine.
Figure 9 illustrates a cross-sectional view of the distal end of the feeding tube apparatus with a magnetic stack extended beyond the distal tip of the catheter.

### DETAILED DESCRIPTION OF THE INVENTION

### I. Feeding Tube Apparatus

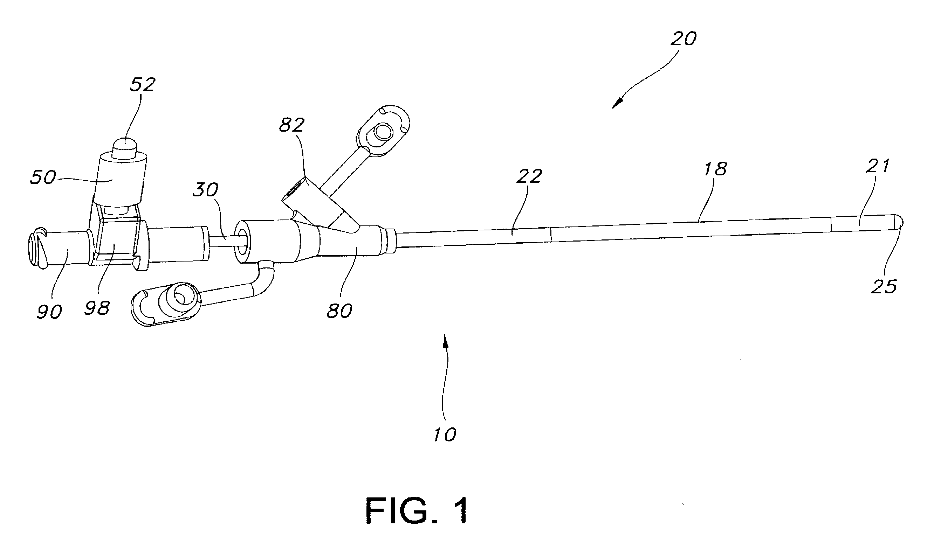

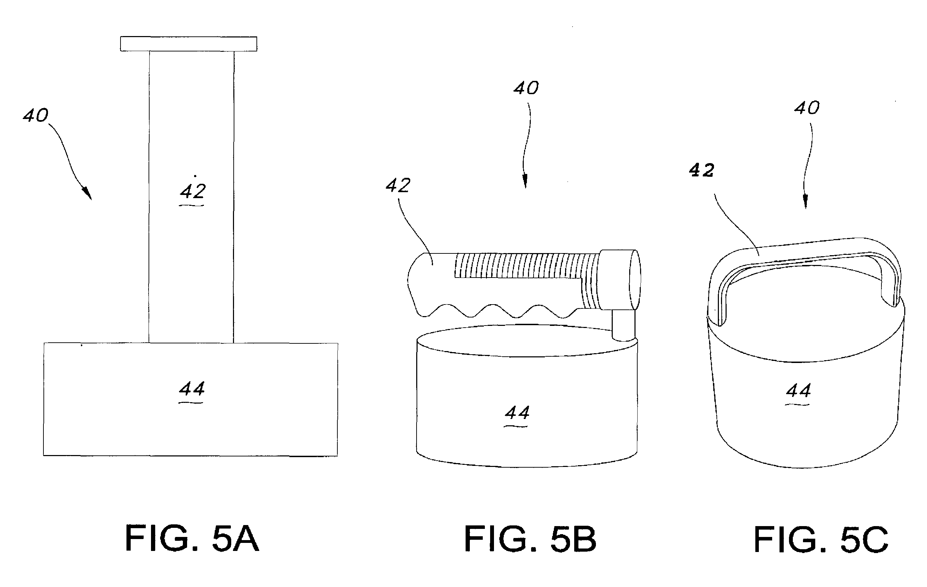

The feeding tube apparatus is depicted in Figures 1 and 2A-C. The feeding tube apparatus (10) contains at least a catheter (20) and a removable stylet (30), where the removable stylet contains one or more magnetic materials (32a-e). The feeding tube apparatus is used in combination with a suitable external magnet (40) (see Figures 5A-C) which a medical practitioner can use to guide the feeding tube apparatus (10) through the intestinal tract. Optionally, the feeding tube apparatus contains additional components, such as a spring wire guide (not shown in figures).

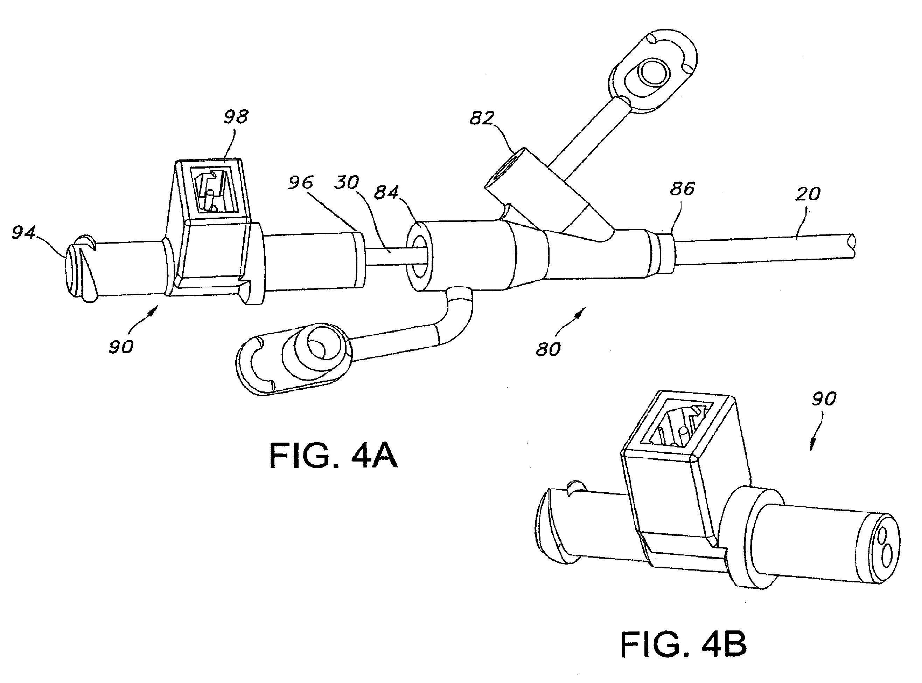

When the feeding tube apparatus is completely assembled, the catheter (20) is connected at its proximal end (22) to a feeding tube hub (80) and the stylet (30) is connected at its proximal end (31) to a stylet hub (90). The feeding tube hub (80) and stylet hub (90) are illustrated in Figures 4A and 4B. The distal end of the stylet hub (90) is insertable into the feeding tube hub (80) and is removable therefrom. The distal end of the stylet hub (90) fits snugly in the feeding tube hub (80).

### A. Catheter

The catheter (20) is a tube with a proximal end (22) and a distal end (24). Generally, the distal tip (25) of the distal end (24) forms an open lumen (26). This allows for the delivery of food from the opening at the distal tip (25) of the catheter. Optionally, the distal tip (25) of the catheter is closed and does not contain an open lumen, but the catheter contains one or more sideholes. In this embodiment, the nutrients are delivered through the sideholes.

The distal tip (25) and the region (21) proximal to the distal tip, which generally corresponds with the region in which the magnetic material(s) is located, is preferably formed of a softer material than the material that forms the rest of the catheter (20). This allows the distal tip (25) and the region (21) proximal to the distal tip to be atraumatic and allows the magnetic material(s) to have a more pronounced effect on maneuverability and guidance than they would if a stiffer material was used. The proximal tip (23) of the proximal end (22) of the catheter (20) also forms an opening (not shown in figures) into which the stylet (30) is placed when it is inserted into the catheter (20).

When the distal end contains an open lumen (26), this allows for the use of a fiberscope, i.e. a flexible, small endoscope, which can be placed through the open lumen to verify the location of the catheter. The use of a fiberscope can eliminate the need for X-rays to be taken to verify the location of the catheter.

### a. Materials

The catheter may be formed of any suitable tubing. Typical tubing materials have a flex modulus ranging from about 3.4 MPa (500 psi) to about 345 MPa (50,000 psi) preferably from 4.8 MPa (700 psi) to 21 MPa (3,000 psi), most preferably about 10 MPa (1,500 psi). Preferably the tubing is dual durometer tubing, with at least two levels of flexibility; where the flex modulus for a first, softer portion is lower than the flext modulus for a second, more rigid portion. In one embodiment, the proximal end (22) comprises a first, relatively soft material, and the distal end (24) is more rigid than the proximal end. Preferably the tubing is relatively soft at the catheter's proximal end (22), at its distal tip (25) and at the region (21) proximal to the distal tip (25), which generally corresponds with the location of the magnetic material(s), and is more stiff in the region (18) between the proximal end (22) and the region (21) proximal to the distal tip (25). The soft material at the proximal end (22), which will be in contact with the patient's throat and nose, causes less irritation to the patient than a stiffer materials. The soft portion of the catheter typically has a flex modulus ranging from about 3.4 MPa (500 psi) to 207 MPa (30,000 psi), preferably ranging from about 5 MPa (750 psi) to 21 MPa (3,000 psi). The stiffer material in the region (18) between the proximal end (22) and the region (21) proximal to the distal tip (25) allows the catheter to have greater pushability and maneuverability during insertion than if a softer material was included in this region (18) of the catheter. The stiffer portion of the catheter typically has a flex modulus ranging from about 10 MPa (1,500 psi) to about 689 MPa (100,000 psi), preferably from about 69 MPa (10,000 psi) to about 645 MPa (50,000 psi). The soft material at the distal tip (25) and at the region (21) proximal to the distal tip (25) allows the catheter to be atraumatic and allows the magnetic material(s) to have a more pronounced effect on maneuverability and guidance of the feeding tube apparatus.

Optionally, the catheter is constructed in whole or in part of a radiopaque material. Suitable materials include polyurethane or silicon tubing. The tubing is preferably comprised of a polymethane for strength. Preferably the polyurethane does not soften or change significantly at body temperature. Examples of suitable polyrrethanes include those available under the tradenames ESTANE® (Lubrizol Advanced Materials, Inc.), PEBAX® (Arkenna France Corp.), PELLETHANE® (Dow Chemical Co.), and CARBOTHANE® (Lubrizol Advanced Materials, Inc.).

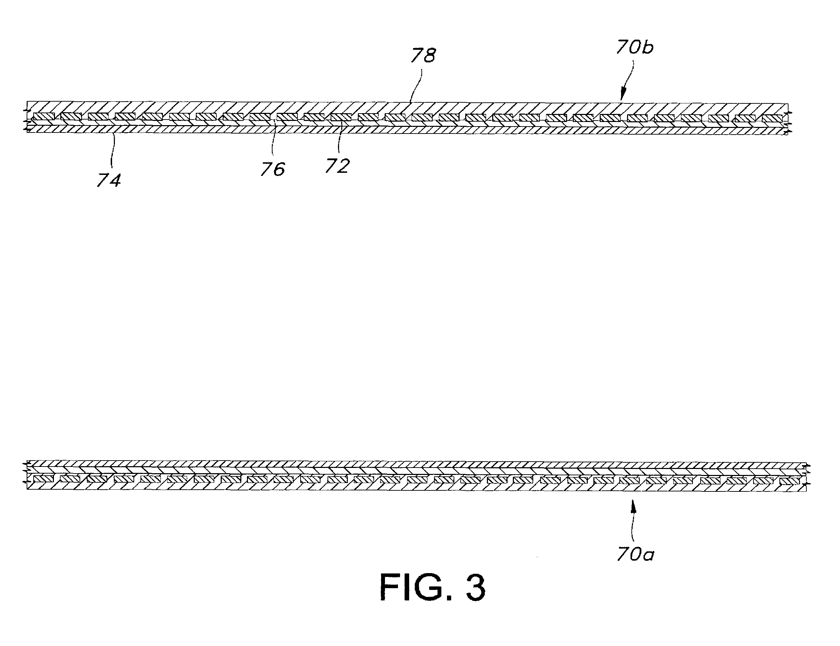

In another embodiment, the walls of the catheter contain a reinforcing material (see Figure 3). In this embodiment, the walls (70a and 70b) of the catheter contain an MRI compatible reinforcing material (72), such as a fiber, monofilament, or non-ferrous metal. This allows the catheter to have a thin wall, while maintaining the desired inner diameter. A reinforcing material also provides kinking- and/or crush-resistance to the catheter. A reinforcing material also allows the catheter to be especially resilient to perforation, thereby facilitating the use of a plunger to purge a clogged catheter without the risk of perforating or damaging the feeding tube, even when the tube is conforming to a tortuous path in the patient's body. In addition, the reinforced construct also allows for reduced internal friction to facilitate stylet removal. Thus comparing a catheter without the reinforcing material with one containing the reinforcing material, the two catheters can have the same inner diameter, but the catheter with the reinforcing material can have a smaller outer diameter than the catheter without a reinforcing material while maintaining the same wall strength. Typical thicknesses for the walls of a catheter without a reinforcing material range from 0.5 mm (0.020 inches) to 0.64 mm (0.025 inches). For catheters with a reinforcing material, typical thicknesses for the walls of the catheter range from 0.2 mm (0.008 inches) to 0.5 mm (0.020 inches). This difference between the wall thicknesses result in a catheter with a reinforcing material with an outer diameter that is one to two French sizes smaller than the outer diameter for a catheter without a reinforcing material, while maintaining the same inner diameter.

Suitable reinforcing materials are stiff, MRI compatible, *i.e*. non-ferrous, materials, such as, polyester, copper, aluminum, non-magnetic stainless steel, or other non-ferrous, stiff, materials. In the preferred embodiment, the reinforcing material is polyester monofilament. However, the reinforcing material can be any stiff, non-ferromagnetic material, such as copper or other monofilament materials. In one preferred embodiment illustrated in Figure 3, the wall (70) of the catheter (30) contains a smooth inner layer (74), a binder layer (76), a reinforcing material (72), and an outer layer (78), The smooth inner layer (74) is typically formed of a material with a low coefficient of friction, i.e. about 0.3 or less, with typical ranges from about 0.1 to 0.4, preferably from 0.1 to 0.2. Suitable materials for the inner layer (74) include FEP (Fluorinated Ethylene Propylene copolymer), PFA (Perfluoroalkoxy), and PTFE (Polytetrafluoroethylene) TEFLON® (E. I. Du Pont de Nemours and Co.). The binder layer (76) is a soft and tacky layer that binds the inner layer (74) with the reinforcing material (72) and, optionally, the outer layer (78). Typical materials have a coefficient of friction, ranging from 0.6 to 1.5, preferably 1.0. A suitable material for the binder layer (76) is 25 D pEBAX®. The material for the outer layer (78) is selected based on the desired stiffness for the catheter, typical materials have a flex modulus ranging from about 3.4 MPa (500 psi) to about 345 MPa (50,000) preferably from 4.8 MPa (700 psi) to 21 MPa (3,000 psi), most preferably about 10 MPa (1,500 psi). Suitable materials for the outer layer (78) include 35D, 40D, or 55D PEBAX®, 75A, 85A, 95A, or 55D CARBOTHANE®, or 80A, 85A, 90A, or 55D PELLETHANE®.

A catheter containing a reinforcing material (72) can be formed using any suitable method, In one embodiment, the catheter is formed by coating the inner layer (74) over a mandrel forming a tube. Then the binder layer (76) is coated over the inner layer (74) forming a coated tube. Next the reinforcing material (72) is spiral wrapped around the coated tube. This is preferably a continuous process. Then an outer layer (78) having the desired stiffness, is pressure-extruded or vacuum-assisted over extruded over the wrapped and coated tube. Then, the mandrel is removed. The resulting catheter (30) contains a reinforcing material (72) in the wall (70) of the catheter. This process produces a thin-walled, kink- and/or crush-resistant catheter.

Alternatively, the reinforced tube can simply contain its reinforcing member (72) on the inner surface and an outer layer (78). Suitable materials for the outer layer (78) include 35D, 40D, or 55D PEBAX®, 75A, 85A, 95A, or 55D CARBOTHANE®, or 80A, 85A, 90A, or 55D PELLETHANE®.

A catheter containing a reinforcing material (72) can be formed using any suitable method. In one embodiment, the reinforcing material (72) is spiral wrapped around the mandrel forming a tube. This is preferably a continuous process. Then an outer layer (78) having the desired stiffness, is pressure-extruded or vacuum-assisted over extruded over the wrapped and coated tube. Then, the mandrel is removed. The resulting catheter (30) contains a reinforcing material (72) in the wall (70) of the catheter. This process produces a thin-walled, kink- and/or crush-resistant catheter.

### b. Dimensions

Any standard diameter and lengths of tubing material may be used to form the catheter. The current standard catheter sizes are referred to as "French" sizes, e.g. size F4 refers to a tube with a 1.35 mm (0.053 inch) outer diameter, F5 refers to a tube with a 1.68 mm (0.066 inch) outer diameter, F6 refers to a tube with a 2.01 mm (0.079 inch) outer diameter, F7 refers to a tube with a 2.34 mm (0.092 inch) outer diameter, F8 refers to a tube with a 3.64 mm (0.104 inch) outer diameter, F10 refers to a tube with a 2.62 mm (0.103 inch) outer diameter, F11 refers to a tube with a 3.63 mm (0.143 inch) outer diameter, and F12 refers to a tube with a 3.96 mm (0.156 inch) outer diameter. In a preferred embodiment, the tubing is a single lumen 2603-80AE PELLETHANE® F11 or F12 tube. The F11 tube has an outer diameter of 3.63 mm (0.143 inches) and an inner diameter of 2.82 mm (0.111 inches); and the F12 tube has an outer diameter of 3.96 mm (0.156 inches) and an inner diameter of 2.95 mm (0.116 inches). However other size tubing is suitable as well In place of single lumen tubing, double lumen tubing or alternative styles may be used. The inner diameter of the tubing (i.e. the diameter of the lumen) should be sufficiently large to allow the fluids and nutrients to pass through the catheter without clogging the catheter. Preferably the inner diameter of the tubing (i.e. the diameter of the lumen) is sufficiently large to allow particles with a diameter of up to 2.79 mm (0.110 inches) to pass through the tubing.

The length of the catheter determines how deep into the gut the feeding tube can be placed for the delivery of fluids and nutrients. Typical lengths for the catheter range from 100 cm to 150 cm, preferably the catheter is at least 125 cm long. In one preferred embodiment, the catheter is 127 cm long. This allows for the nutrients to be delivered deep into the bowel and thereby prevent reflux. Catheters that are at least 100 cm long prevent the patient from inadvertently removing the feeding tube after placement in the stomach such as through standard movements.

### c. Sideholes

In addition to the openings at the proximal and distal ends of the catheter, the catheter optionally contains one or more holes along the wall of the catheter, referred to herein as "sideholes" (28). Preferably the sideholes are located as close to the distal tip (25) as possible without compromising the strength of the tubing and interfering with the magnetic material(s) (32) and reed switch assembly (60). In one embodiment, the sideholes are located in the catheter in the region (18) between the proximal end (22) and the region (21) proximal to the distal tip (25) of the catheter (*see e.g.,* Figure 2A). In another embodiment, the sideholes may also be located within the region (21) proximal to the distal tip (25) of the catheter (*see e.g.,* Figures 2B and 2C).

In one embodiment, the distal end of the catheter does not contain an opening, but the catheter contains one or more sideholes. In this embodiment, the nutrients are delivered through the sideholes.

The sideholes ensure that, even if the feeding tube is lodged against a wall in a patient's body, aspirating the catheter will not create a suction situation and potentially damage internal tissues or walls.

Moving along the length of the catheter starting at the distal tip (25), the magnetic material(s) (32) is located in the stylet in a region that generally corresponds with the region (21) proximal to the distal tip (25), the reed switch assembly (60) is located in the stylet proximal to the magnetic material(s) (32)., In one embodiment, the sideholes (28) are located in the catheter in a region that is generally proximal to the location of the reed switch assembly (60) (*see e.g.* Figure 2A). In another embodiment, the sideholes are located within the region (21) proximal to the distal tip (25) of the catheter (*see e.g*., Figures 2B and 2C).

In one embodiment, the catheter contains two sideholes (28a and 28b). The sideholes are typically oval or circular in shape and typically have dimensions ranging from 0.25 mm (0.010 inches) to 2.79 mm (0.110 inches), preferably the sideholes have diameter of about 1.27 mm (0.050 inches).

### B. Stylet

The stylet (30) contains a proximal end (31) and a distal end (34), when the distal end terminates in a distal tip (35). As illustrated in Figure 2A, moving from the center of the stylet (30) towards the distal tip (35) of the style (30), the distal end (34) of the stylet contains a reed switch assembly (60), which is attached to a spacer (37), which is attached to a magnetic material (32). Typically the distal end has a length tanging from 1 to 6 inches, preferably the distal end is 3 inches long. The magnetic material(s) or magnet stack may terminate at the distal tip (35) of the stylet.

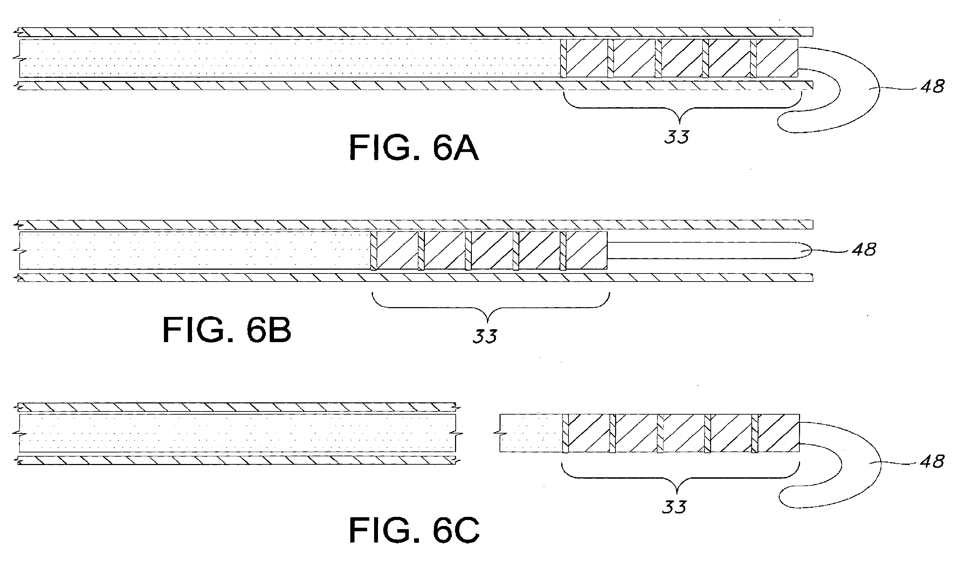

Optionally, the stylet contains an additional element, such as a J-tip spring wire guide (48) or a pigtail-shaped spring wire guide, which is attached to the magnetic material or magnet stack and terminates at the distal, end of the stylet (see Figures 6A-C). When in use, the J-tip facilitates passage of the stylet through anatomical structures, particularly the duodenum, without catching on any anatomical pockets. Alternatively, instead of a J-tip, the stylet could contain a pigtail-shaped spring wire guide which terminates at the distal tip (35) of the stylet, to achieve the same effect as a J-tipped spring wire guide.

### a. Materials

The stylet (30) is typically in the form of a tube and is formed of material that is more rigid than the catheter material, Typical flex modulus values for the stylet range from 862 and 2413 MPa (125,000 and 350,000 psi), preferably from about 1206 MPa (175.000 psi) to 1123 MPa (250,000 psi), most preferably 1379 MPa (200,000 psi). Suitable materials include polycarbonate, polyether ether ketone (PEEK), nylon 6/6, stiff polyurethanes such as 75D PELLETHANE®,or another rigid material. The stylet provides column strength to the feeding tube apparatus and facilitates guidance of the catheter during placement in the intestinal tract.

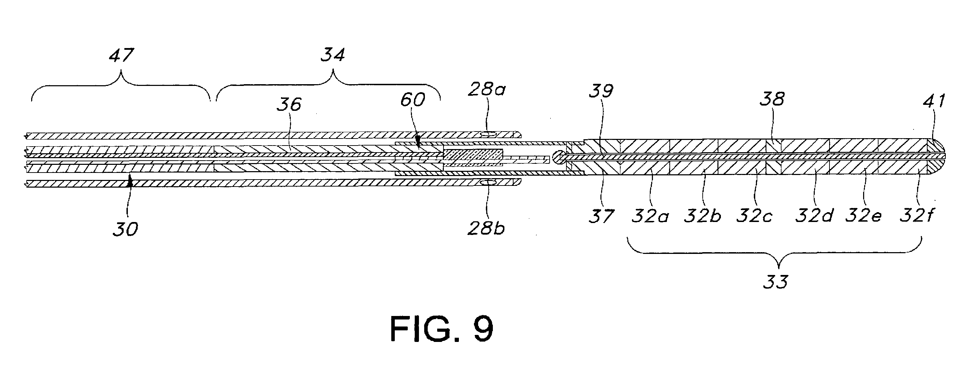

Preferably the stylet (30) is a formed from a dual durometer material, i.e. a material with at least two levels of flexibility (*see e.g*. Figure 9). In a preferred embodiment, the stylet is formed from two different materials that are joined together, where one material is a stiffer material than the other material. Typical flex modulus values for a first, soft portion of the stylet range from about 172 MPa (25,000 psi) to about 862 MPa (125,000 psi), preferably from 172 MPa (25,000 psi) to 517 MPa (75,000 psi). Typical flex modulus values for a second, more rigid portion of the stylet range from about 862 and 2758 MPa (125,000 and about 400,000 psi) preferably about 172d- (250,000 psi). Preferably the stiffer material is used for the majority of the length of the stylet, while the more flexible material is used for only the distal end (34) of the stylet. In another embodiment, the stylet is formed from a first material and contains a second material as a coating over the first material for a portion of the length of the stylet, preferably for the majority of the length of the stylet, most preferably only the distal end (34) of the stylet is not covered by the second material. In one embodiment, the stylet is formed from nylon, and the nylon is coated with a polyester shrink wrap (e.g. Advanced Polymers, Inc.) along the majority of the length of the stylet, with the exception of the distal end (34), which contains only the nylon material and does not contain the shrink wrap coating. In this embodiment, the distal end is more flexible than the remainder of the stylet. In one preferred embodiment, the flexible portion (36) of the stylet is 76 mm (3.0 inches) in length. In other embodiments, the flexible portion (36) of the stylet may be from 1 inch to 6 inches in length. The stylet is more rigid in the region (47) that starts at the proximal end (31) of the stylet where it connects to the stylet hub (90) and ends where the flexible portion begins. Typically, the flexible portion typically begins immediately adjacent and proximal to the reed switch assembly.

### b. Dimensions

In one embodiment, the stylet (30) is long enough to extend along the length of the catheter (20), but not beyond the open lumen (26) at the distal tip (25) of the catheter. In another embodiment, the stylet (30) is long enough to extend along the length of the catheter (20) and beyond the open lumen (26) at the distal tip (25) of the catheter, which allows the catheter to track over a stylet already in place in the desired location. Thus the stylet can guide the feeding tube to its desired location, by passing the feeding tube over the stylet until it reaches the desired placement location.

Typical lengths for the stylet range from about 127 cm, which generally corresponds with the length of the catheter, to a length greater than the length of the catheter, such as about 175 cm, which allows for the stylet to extend beyond the distal tip of the catheter. Preferably, the stylet is about 127 cm long.

The outer diameter of the stylet is selected based on the inner diameter of the catheter. The outer diameter of the stylet is less than the inner diameter of the catheter so that the stylet can easily slide into and out of the catheter, as desired. By way of example, for catheters formed using 11 FR or 12 FR tubing, the stylet may have an outer diameter from 0.76 to 2.72 mm (0.030 to 0.107 inches).

As used herein "magnetic material" refers to both magnets and magnetically attractive materials.

As used herein "magnet" refers to a material that both produces its own magnetic field and responds to magnetic fields. Magnets include permanent magnets, which remain magnetized, and impermanent magnets, which lose their memory of previous magnetizations. Magnets include but are not limited to the following materials: Neodymium (Rare Earth), Samarium Cobalt (Rare Earth), Ceramic (Ferrite), and Alnico (Aluminum Nickel Cobalt).

As used herein "magnetically attractive material" refers to materials that do not produce a magnetic field, but that are attracted to a magnetic field or to each other when in the presence of a magnetic field, and include paramagnetic materials. Magnetically attractive materials include but are not limited to the following materials: iron, preferably iron coated with Teflon, polyimide, or parylene, or another suitable material to make it biocompatible, and steel.

As used herein "spacer" refers to a flexible material that neither produces its own magnetic field nor responds to magnetic fields. Materials that are useful for forming spacers include the materials used to form the catheter. Typical materials have a flex modulus ranging from about 3.4 MPa (500 psi) to about 375 MPa (50,000 psi), preferably from 4.8 MPa (700 psi) to 21 MPa (3,000 psi), most preferably about 10 MPa (1,500 psi). Examples of suitable materials include but are not limited to, any flexible plastic, such as one formed from a soft polyurethane or silicon; examples include PEBAX®, PELLETHANE®, CARBOTHANE®, all in the 75A to 55D hardness range or therabout.

The distal end (34) of the stylet (30) contains one or more magnetic materials (32), and preferably contains a plurality of magnetic materials, optionally in combination with one or m.)r6 spacers, referred to herein as a "magnet stack" (33). The one or more magnetic materials may be in groups (39) of stacked magnetic materials, where each group (39) is separated from the next group (39) with a spacer.

Optionally, the distal end (34) of the stylet (30) contains a magnet stack (33) or a magnetic material (32) in the form of a continuous and flexible coil or wire strand. The length of the magnetic material (32) or magnet stack (33) can be any suitable length for obtaining the necessary magnetic field between the external magnet (40) and the magnetic material(s) (32) or magnet stack (33) in the stylet. Typical lengths for the magnetic materials (32), preferably when the magnetic material is a magnet, range from 0.25 mm (0.01 inches) to 12.5 mm (0.5 inches) preferably from 2.5 to 7.5 mm (0.1 to 0.3 inches), or from 5 to 5.7 mPa (0.2 to 0.225 inches). However, magnetically attractive materials may have the same dimensions as magnets. In one preferred embodiment the length of each magnetic material is 5 mm (0.200 inches).

Typical lengths for the magnet stack (33) range from 6.4 mm (0.25 inches) to 51 mm (2.0 inches), preferably the magnet stack (33) is about 38 mm (1.5 inches) long. Typical lengths for the magnetic material when the magnetic material is a magnetically attractive material range from 6.4 mm (0.25 inches) to 51 mm (2.0 inches). preferably about 38 mm (1.5 inches).

In a preferred embodiment, the magnet stack (33) contains two or more stacked magnets with each magnet having the same dimensions. Preferably the magnets are connected to each other by a connecting element, such as a wire (45). In a preferred embodiment, the wire (45) extends through a hole in the center of each magnet (*see* Figures 2A-C), The connecting element is preferably suitably affixed to the magnet stack to ensure that it remains connected with the magnet stack. For example, the wire (45) may terminate at the distal end of the magnet stack (33) with a material that is wider than the hole in the magnet stack, such as in the form of a bead or a bullet-nose (41). In a particularly preferred embodiment, the bead is formed using a U/V adhesive material, which solidifies to form the bead when it is exposed to ultraviolet light. For additional flexibility each magnetic material (32) in a magnet stack may be separated from the proximal magnetic material or each group (39) of stacked magnetic materials may be separated from the proximal group with a flexible spacer (38a-d), such as one formed from a soft polyurethane or silicon. In yet another preferred embodiment, the magnet stack may contain groups of magnets separated by a spacer. This facilitates movement of the magnet stack through the patient's body, particularly along curves.

The magnet stack may contain two or more groups of magnets, where each group contains the same number of magnets, such as ranging from two to five magnets, preferably two magnets, where each group is separated from the next group by a flexible spacer (38). For example, as shown in Figure 9, the magnet stack may contain two groups of magnets, where each group contains three magnets, and where the first group is separated from the second group by a spacer (38).

A cross-sectional view of the preferred embodiment is illustrated in Figure 2B. In the preferred embodiment the magnet stack (33) contains three groups (39a - c) of stacked magnet materials (32a-e) with each group containing two magnetic materials (e.g. 32 a, 32b) and where each group (e.g. 39a) is separated from the proxima group (e.g. 39b) by a spacer (e.g. 38a) (*see e.g.* Figure 2B).

In a particularly preferred embodiment, Neodymium, most preferably grade N50, is used in each magnetic material in the magnet stack. Preferably the length of each magnetic material is 5 mm (0.200 inches). Preferably PEBAX®, most preferably 35D PEBAX®, is used in each spacer between the groups of magnetic materials. Preferably the length of each spacer within the magnet stack is 1.3 mm (0.050 inches). In the preferred embodiment, the distal tip of the stylet contains a U/V adhesive material, most preferably the U/V adhesive material forms a bullet-nose tip (41).

The diameter of the magnetic material(s) is selected based on the inner diameter of the catheter. The outer diameter of the magnetic material(s) is less than the inner diameter of the catheter so that the magnetic material(s) can easily slide into and out of the catheter, as desired. In a preferred embodiment, the diameter of the magnetic materials is greater than the diameter of the stylet, and less than the inner diameter of the catheter. This allows the magnetic material(s), preferably in the form of a magnet stack (33), to provide the greatest magnetic force for the area within the catheter, and seals the opening (26) at the distal tip (25) when the stylet is inside the catheter. By sealing the distal tip, when aspiration occurs, it will generally only occur through the sideholes (28a and 28b), and will not also occur at the distal tip (25). By way of example, for catheters formed using 11 FR or 12 FR tubing, the magnet(s) may have an outer diameter 1.3 mm (0.050 inches) to 3.2 mm (0.125 inches), with a preferred diameter of about 2.5 mm (0.1 inches).

In one embodiment, the magnetic materials are in the form of a stranded wire or a coiled assembly such as a helically wrapped wire. This embodiment is particularly preferred for magnetically attractive materials. Preferably the magnetically attractive material is iron, more preferably in the form of galvanized iron wire. The stranded wire and the coiled assembly construct should have suitable flexibility so that no spacers are needed. This embodiment allows for a continuous magnetically attractive material at the distal tip of the stylet.

One embodiment of the device which contains the magnetic materials in the form of a stranded wire or a coiled assembly is shown in Figure 2C. In this embodiment, the stylet contains a reed switch assembly (60), which is located at the distal end (34) of the stylet and is attached to a spacer (37), which is attached to the magnetic material (32) in the form of a stranded wire, or to a coiled assembly such as a helically wrapped wire.

The diameters for a magnetically attractive material in the form of coil or stranded wire are the same as the suitable diameters listed above for the magnetic materials in general. Thus typical diameters range from 1.3 mm (0.050 inches) to 3.2 mm (0.125 inches), with a preferred diameter of about 2.5 mm (0.1 inches). For example, the coil may be formed using a 0.5 mm (.020 inch) diameter core with four (4) layers of 0.25 mm (0.010 inch) diameter iron wire. Thus the total diameter coil is about 2.5 mm (0.1 inches). Alternatively, the magnetically attractive material may be in the form of a stranded wire. A 7 x 19 or similar stranded wire may be used to achieve a diameter of about 2.5 mm (0.1 inches)

### d. Reed Switch Assembly

The stylet (30) contains a reed switch assembly (60) to ensure that an indicator (52) properly indicates when the magnetic force between the magnetic material(s) (32) or magnetic stack (33) in the stylet and the external magnet (40) is sufficiently strong to move the catheter (20) along the intestinal tract using the external magnet (40).

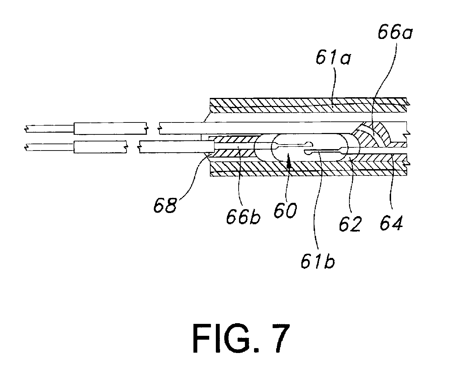

An example of a suitable reed switch assembly (60) is described in U.S. Patent No. 6,126,647 to Posey and is illustrated in Figures 2 and 7.

The reed switch assembly (60) contains reeds (61a and 61b) sealed in a glass envelope (62), which is disposed within a metal housing (64). The leads (66a and 66b) are soldered to the external portions of the reeds (61a and 61b), and the metal housing (64) of the reed switch assembly (60) is affixed to the stylet (30) and thereby connects the reed switch assembly (60) the spacer (37), the magnetic material(s) (32), which are preferably in the form of a magnet stack (33), and, optionally the spring wire guide, In a preferred embodiment, the magnetic material(s) (32), optionally in the form of a magnet stack (33), is coated with a biocompatible coating that also provide a lubricious surface, such as parylene, to facilitate easy sliding of the stylet in and out of the feeding tube. Alternatively, a heat shrink tube, such as polyester shrink, can also be employed to encapsulate this assembly and thereby ensure that the reed switch assembly (60) the spacer (37), the magnetic material(s) (32) or magnet stack (33), and, optionally the spring wire guide, do not separate from the stylet (30). Heat shrink tubing also provides a lubricious surface between the inner surface of the catheter and the stylet (30) to facilitate insertion and removal of the stylet (30) from the catheter (20).

For an external magnet (40) having a magnetic flux field of about 0·03 Tesla (300 Gauss) at a distance of 100 mm (4 inches) from the external magnet (40), the reeds (61a and 61b) in the reed switch assembly (60) will contact each other, thereby actuating the indicator (52), when the external magnet (40) is within 89-131 mm (3.5 to 5.0 inches) of the reed switch (60). In one preferred embodiment, the reeds contact each other, thereby actuating the indicator (52), when the external magnet (40) is within about 4 inches of the reed switch assembly (60). The indicator (52) produces a signal when it is actuated. The signal indicates that the magnetic force between the feeding tube catheter and the external permanent magnet is strong enough to use the external magnet to direct the feeding tube catheter to the desired location.

The dimensions of the spacer (37) are selected based on the distance between external magnet (40) and the reed switch (60) required to actuate the indicator (52). For example, in one embodiment, spacer (37) between the reed switch (60) and the proximal end of the magnetic material(s) (32) or magnet stack (33) is at least 2 mm long. A feeding tube apparatus (10) designed in this manner will typically produce a signal when the external magnet (40) is within about 100 mm (4 inches) of the reed switch (60).

Although the stylet (30) is illustrated herein as containing a normally open reed switch assembly (60) disposed within a metal housing (64), other reed switches, such as those that are normally closed, may be used. A single-pole, single-throw (SPST), normally-open reed switch (also referred to as a Form "A" reed switch) is illustrated herein. Single-pole, single-throw (SPST), normally-closed reed switches (also referred to as Form "B" reed switches), single-pole, double-throw (SPDT), and break-before-make reed switches (also referred as Form "C" reed switches) are known in the art and may be used in place of the reed switch (60) depicted in Figures 2A-C, 7 and 9. Regardless of the particular type of switch used, e.g. Form A, Form B, or Form C, each type of reed switch can be used as described herein.

### e. Spring Wire Guide

In addition to containing the magnetic material (32) or magnet stack (33), the distal end (34) of the stylet (30) may also contain a spring wire guide (48). The spring wire guide may be a J-wire, a pigtail or a straight spring wire guide. An example of this embodiment is provided in Figures 6A-C. In this embodiment, moving along the distal end (34) towards the distal tip (35), the distal end (34) of the stylet (30) contains the reed switch assembly (60), which is attached to a spacer (37), which is attached to a magnetic material (32) or magnet stack (33), which is attached to a J-wire spring wire guide (48). In this embodiment, the J-wire spring wire guide is located at the distal tip (35) of the stylet (30). Alternatively, a straight or pigtail spring wire guide (not shown in figures) could be used in place of the J-wire.

The spring wire guide facilitates guiding the catheter through the intestinal tract. In this embodiment, the spring wire guide is particularly useful at guiding the catheter after it passes through the pyloric valve, especially as it advances deep into the duodenum.

A pigtail can be formed at the distal tip (35) of the stylet to achieve the same effect as the J-wire. A pigtail is preferably formed from a flexible polyurethane, such as PELLETHANE®, PEBAX® or ESTANE®, such that it can easily be straightened when pulled into the feeding tube, and once extended beyond the feeding tube, can easily resume its pigtail shape to facilitate advancement of the stylet through the patient's body, especially in the duodenum.

### f. Signal Generator

A signal generator (50) attaches to the proximal end of the stylet via a stylet hub (90).

Typically the catheter (20) is inserted into a feeding tube hub (80), which contains one or more ports (82) to allow for aspiration or delivery of medications. The feeding tube hub contains an opening at each end (84 and 86) and is hollow throughout the length of the hub (80). The catheter (20) exits the feeding tube hub at the distal end (86) of the feeding tube hub.

The proximal end (84) of the feeding tube hub attaches to the distal end (96) of a stylet hub (90). The stylet hub (90) contains an opening at each end (94 and 96) and is hollow throughout the length of the stylet hub. The stylet (30) exits the stylet hub (90) at the distal end (96) of the stylet hub (90) and extends inside and along the length of the catheter (20). The stylet hub (90) also contains a port (98) for the signal generator (50). The port (98) preferably contains a socket with which an LED plug can connect and thereby provide a visual signal when the external magnet (40) is at an appropriate distance from the magnetic material(s) (32). The signal generator (50) is electronically connected to the reed switch assembly (60) via the port (98).

The signal generator (50) includes a power source, such as a battery, (not shown in figure) which supplies power to an indicator (52) when the reeds (61a and 61b) close in response to a magnetic field supplied by the external magnet (40) which is sufficiently strong to permit manipulation of the distal end (24) of the catheter (20) by movement of the external magnet (40). Thus, when the external magnet (40) is at the minimum distance required to supply a sufficiently strong magnetic field to allow the external magnet (40) to manipulate of the distal end (24) of the catheter (20), the reeds (61a and 61 b) in the reed switch assembly (60) contact each other and thereby actuate the signal generator (50).

The indicator (52) in the signal generator (50) can produce any suitable signal that can be distinguished by a user, such as a light, a vibration, a sound, or a digital readout.. In the preferred embodiment, the indicator is a light, such as a light emitting diode (LED).

### C. Optional Components

Optionally, the feeding tube apparatus (10) contains a spring wire guide that is not attached to the stylet (not shown in figures). The spring wire guide may be a J-wire or a straight spring wire guide. In this embodiment, after the stylet (30) is removed from the catheter (20), the spring wire guide can be placed in the catheter (20) until it protrudes from the opening (26) at the distal end (25) of the catheter. Then the spring wire guide can be used to facilitate guidance of the catheter as it advances through the intestinal tract.

Optionally, the feeding tube apparatus contains a plunger (not shown in figures) that can clear debris that collects in the catheter to eliminate the need to remove catheter and replace with another one. In this embodiment, after the stylet (30) is removed from the catheter (20), the plunger can be placed in the catheter (20) to remove any debris that is obstructing the delivery of nutrients and/or medicine to the patient, and/or preventing aspiration.

Optionally the distal end of the stylet contains a pH sensor probe, connected to a digital pH meter at the catheter proximal end. This allows one to measure the pH of the surrounding environment around the catheter distal end as the feeding tube apparatus is maneuvered through the patient to help determine when the feeding tube apparatus reaches the desired location for placement.

### II. Kits for the Feeding Tube apparatus

A kit containing the feeding tube apparatus described above and an external magnet may be provided.

### a. External Magnet

The external magnet (40) can have any suitable shape or size that allows manipulation by the healthcare provider. Figures 5A, B and C depict three representative designs for the external magnet. The external magnet typically has a handle (42) attached to abase magnet (44). The handle may be affixed perpendicularly to the base magnet (44), as shown in Figure 5A. Alternatively, the handle may be attached to the base magnet (44) so that it is parallel with the base magnet (44), as shown in Figures 5B and 5C.

The base magnet may have a wide range of dimensions and shapes. In a preferred embodiment, the base magnet contains a surface distal to the handle which is flat. This surface is designed to be placed in contact with a patient's body. In one preferred embodiment, the base magnet is in the shape of a cylinder.

Typical diameters for the base magnet range from about 25 mm (1 inch) to about 121 mm (5 inches) preferably from about 1-6 mm (3 inches) to about 102 mm (7 inches) Typical heights for the external magnet (i.e. both the handle and the base magnet) range from about 16 mm (3 inches) to about 152 mm (6 inches) preferably from about 16 mm (3 inches) to about 102 mm (4 inches).

to about 102 mm (4 inches). The preferred material for the base magnet is Nedoymium N50 grade, which can be used to form a small and light weight magnet that provides the highest practical magnetic flux for its size and weight.

### i. Electromagnet

In one embodiment, the external magnet is an electromagnet instead of a permanent magnet In this embodiment, the electromagnet is preferably attached to a modular power supply via a tether. The tether, in addition to carrying the conductors, may provide conduits for water or gas circulation to cool the electromagnetic probe and leads. For safety purposes, the tether preferably has a braided ground wire. Thus if the conductive leads carrying the high current to drive the electromagnet were in any way exposed, the tether would short to ground, and the power supply would be immediately shut down.

Benefits to using an electromagnet in the external magnet in place of a permanent magnet include, allowing the magnetic field to be further localized (smaller probe focuses the energy better), resulting in a more focused and stronger magnetic field. This facilitates guiding the feeding tube to its desired location. Additionally, an external magnet that contains an electromagnet can be much lighter than an external magnet containing a permanent magnet, such as having a weight from about 0.45 to 1,36 kg (1 to 3 pounds), to develop the same magnetic field. Further, the electromagnet is switchable, so that it can be easily turned off when not it is in use. This prevents accidental movement of the feeding tube apparatus out of its desired location after placement in its desired location-due to movement of the external magnet.

Another benefit of an electromagnet is that it may eliminate the need for the reed switch on the stylet, further reducing cost and complexity of the stylet construction. This is possible since an electromagnet can monitor and indicate the presence of another ferrous material that is being attracted to it As such, a numeric or graphical indicator on the electromagnet power supply could provide all attraction indicator. With this construct, it would then not be necessary to have a reed switch in the stylet, while still providing an indication as to the presence and localization of the stylet tip to the electromagnetic probe.

### c. Optional components

Optionally the kit also includes one or more additional components that assist the medical practitioner in use of the feeding tube apparatus. Suitable additional components include a syringe, preferably a 60 CC syringe; one or more towels; one or more cups, preferably at least two cups; disposable gloves; Xylocaine gel (e.g. 2% Xylocaine gel); tape; gauze; disposable magnet covers; spring wire guide, and/or pH paper. Optionally, the kit contains a plunger or obturator that can clear clogs in the catheter to eliminate the need to remove the catheter and replace with another one. Optionally, the kit contains a spring wire guide that can be inserted into the catheter after the stylet is removed.

### III. Method of using Apparatus

The distal tip (25) of the catheter is introduced into the naris of the patient's nose and advanced by the continued application of a compressive force to the catheter forcing the distal tip (25) to the back portion of the patient's head and into the esophagus. As is common, the passageway of the esophagus affords ample guidance to the distal tip (25) whereupon it enters the body portion of the stomach.

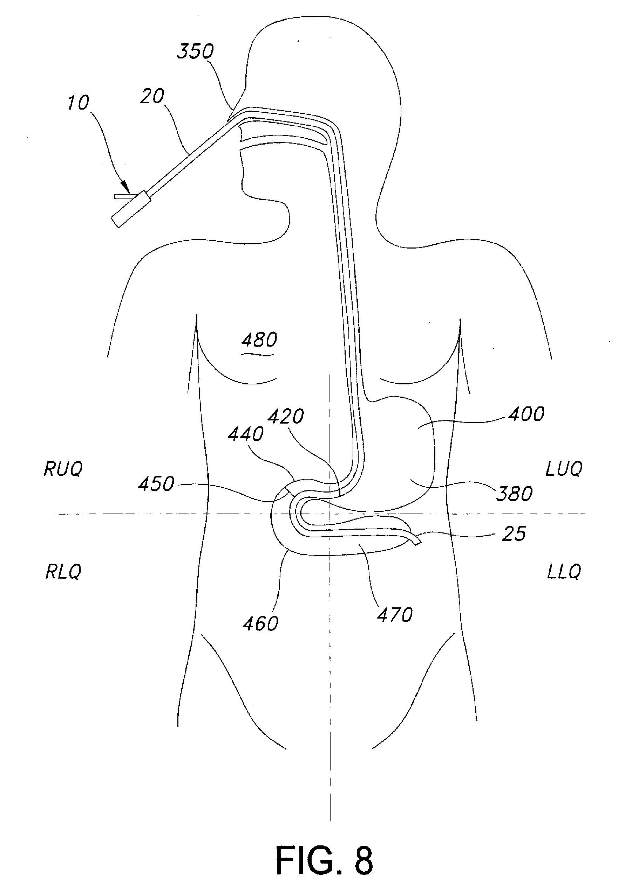

Figure 8 is an illustration showing the path of the catheter (20) within anatomical quadrants of the small intestine. The catheter passes through the stomach (380) to the distal duodenum (470) to allow for feeding to occur in the distal duodenum (470), and thereby prevent aspiration of fluids to the stomach.

After the catheter is placed in the desired location, the stylet is removed. The catheter can remain in place when the patient undergoes diagnostic tests, such as MRI imaging since the magnet or magnet stack was removed from the catheter when the stylet was removed.

The stomach (380) has a generally J-shaped configuration extending with generally its largest transverse anatomical size at about the cardiac orifice, the entrance site to the stomach (380), and then proceeding in the direction at which the stomach (380) functions to advance bolus, the transverse dimension of the stomach narrows, and at an angular notch (420) which is generally at the border between the left upper quadrant (LUQ) and the right upper quadrant ("RUQ"). From the annular notch (420) there commences a smaller transverse dimension at the pyloric part (440) typically residing in the right upper quadrant together with pyloric sphincter (450). The pyloric sphincter (450) is a muscular controlled closure which will dilate as when a bolus comes into contact with the sphincter. Beyond the sphincter, a bolus passes into the duodenum portion (460) that extends to the right lower quadrant ("RLQ"), and then extends in a general horizontal direction into the left lower quadrant ("LLQ") where the distal duodenum (470) of the small intestine is located.

The catheter (20) of the feeding tube apparatus (10) described herein can be directed through the intestinal tract to the desired location for delivering nutrients using the external magnet (40). The distal tip (25) of the catheter (20) is introduced into the naris (350) of the nose and advanced by the continued application of a compressive force to the catheter forcing the distal tip (25) of the catheter (20) back to the backward portion of the patient's head and into the esophagus. Typically, the passageway of the esophagus provides ample guidance to the distal tip (25) of the catheter (20) whereupon it enters the body portion of the stomach (380) at the lower portion of the fundus. The movement of the distal tip (25) along the esophagus and into the body of the stomach (380) occurs within the left upper quadrant ("LUQ") of the patient's body, as indicated in Figure 8. Then the external magnet (40) is applied to the patient's body within the minimum distance required to create a magnetic field between the external magnet (40) and the magnetic material(s) (32) located at the distal end (34) of the stylet (30) that is sufficiently strong to allow the external magnet (40) to guide the feeding tube apparatus (10) through the stomach (380), the annular notch (420), the pyloric sphincter (450), into the duodenum portion (460), and into the distal duodenum (470) of the small intestine.

In one embodiment, the stylet (30) contains a spring wire guide (48) at its distal tip (35). In this embodiment, the catheter may be advanced through the stomach, using the external magnet (40) as a guide until the catheter passes through the pyloric sphincter (450) with the distal tip (35) of the stylet (30) located inside the catheter, at the distal tip (25) of the catheter (20), as shown in Figure 6B. After the catheter passes through the pyloric sphincter (450), the spring wire guide (48) may be extended beyond the distal tip (25) of the catheter, as shown in Figure 6A. Optionally, both the spring wire guide and the magnetic material(s) (32) are extended beyond the distal tip (25) of the catheter, as shown in Figure 6C. By extending the stylet in this manner, the spring wire guide can be used to help navigate the catheter through the curvy the duodenum portion (460) to the desired location.

In another embodiment, the stylet does not contain a spring wire guide at its distal tip (35). In this embodiment, the catheter may be advanced through the stomach, using the external magnet (40) as a guide until the catheter passes through the pyloric sphincter (450) with the distal tip (35) of the stylet (30) located inside the catheter, and at the distal tip (25) of the catheter (20), as shown in Figure 2A. After the catheter passes through the pyloric sphincter, the stylet may be removed from the catheter and, optionally, a spring wire guide may be placed in the catheter and extended beyond the distal tip (25) of the catheter (not shown in figures). The spring wire guide may be a J-wire or a straight spring wire guide. By extending the spring wire guide in this manner, the spring wire guide can be used to help navigate the catheter through the curvy the duodenum portion (460) to the desired location.

Unless defined otherwise, all technical and scientific terms used herein have the same meanings as commonly understood by one of skill in the art to which the disclosed invention belongs.

Those skilled in the art will recognize, or be able to ascertain using no more than routine experimentation, many equivalents to the specific embodiments of the invention described herein. Such equivalents are intended to be encompassed by the following claims.

## Claims

1. A feeding tube apparatus (10) comprising a catheter (20) and a magnetically guidable, removable stylet (30), wherein the stylet (30) comprises a proximal end (31) formed of a first material having a flex modutus value of about 862 MPa (125,000 psi) to about 2758 MPa (400,000 psi) and a distal end (34) formed of a second material different than the first material and having a flex modulus value of up to 862 MPa (125,000 psi) wherein the distal end (34) is more flexible than the proximal end (31), wherein the stylet (30) is insertable into the catheter (20) and removable therefrom, wherein the distal end of the stylet (30) comprises more than one magnetic material (32) and a spacer (39) between each magnetic material (32), wherein the spacer (39) comprises a flexible material having a hardness up to approximately 55D,
wherein the magnetic materials (32) are such as to create a magnetic field between an external magnet (40) and the magnetic materials (32) that is sufficiently strong to.allow he external magnet to guide the style (30) inside a patient's body when the external magnet (40) is about 100 mm (4 inches) from the magnetic materials (32),
wherein the distal end (34) terminates with a distal tip (35), and
wherein the catheter (20) is magnetic resonance imaging (MRI) compatible.

2. The feeding tube apparatus of Claim 1, wherein the style (30) further comprises a reed switch assembly (60).

3. The feeding tube apparatus of Claim 1, wherein the magnetic material (32) is selected from the group consisting of magnets and magnetically attractive materials.

4. The feeding tube apparatus of any of Claims 1 to 3, wherein the style (30) further comprises a spring wire guide (49) distal to the magnetic materials (32), wherein the spring wire guide (49) terminates at the distal tip (35) of the stylet (20).

5. The feeding tube apparatus of any of Claims 1 to 4, further comprising a signal generator (50) connected to the proximal end (31) of the style (30), wherein the signal generator (50) comprises an indicator (52) capable of emitting a signal selected from the group consisting of light, sound, vibration, and digital readout.

6. The feeding tube apparatus of Claim 5, wherein the indicator (52) is a light emitting diode (LED).

7. The feeding tube apparatus of any preceding claim, wherein the style (30) is formed from a dual durometer material.

8. The feeding tube apparatus of any preceding claim, wherein the stylet (30) comprises a pH sensor at the distal tip (35) of the stylet (30).

9. The feeding tube apparatus of any preceding claim, wherein the wall (10) of the catheter (20) comprises an MRI compatible reinforcing material.

10. The feeding tube apparatus of any preceding claim, wherein the wall (10) of the catheter (20) comprises a smooth inner layer (14), a binder layer (16), a reinforcing material (12), and an outer layer (13).

11. The apparatus of any preceding claim, wherein the catheter (20) is formed from a material selected from the group consisting of polyurethane and silicon tubing.

12. The apparatus of any preceding claim, wherein the magnetic materials (32) are such as to create a magnetic field between an external magnet that has a magnetic flux field of about 0.03 Tesla (300 Gauss) at a distance of 100 mm (4 inches) from the external magnet (40) and the magnetic materials (32), and wherein the magnetic field is sufficiently strong to allow the external magnet (40) to guide the style (30) inside a patient's body when the external magnet (40) is about 100 mm (4 inches) from the magnetic materials (32).

## Patentansprüche

1. Magensondenvorrichtung (10), umfassend einen Katheter (20) und einen magnetisch führbaren, entfernbaren Führungsstab (30), wobei der Führungsstab (30) ein proximales Ende (31), welches aus einem ersten Material gebildet ist, welches einen Elastizitätsmodulwert von ungefähr 862 MPa (125000 psi) bis ungefähr 2758 Mpa (400000 psi) aufweist, und ein distales Ende (34) umfasst, welches aus einem von dem ersten Material verschiedenen zweiten Material gebildet ist und welches einen Elastizitätsmodulwert von bis zu 862 MPa (125000 psi) aufweist, wobei das distale Ende (34) flexibler als das proximale Ende (31) ist, wobei der Führungsstab (30) in den Katheter (20) einführbar und daraus entfernbar ist, wobei das distale Ende des Führungsstabs (30) mehr als ein magnetisches Material (32) und einen Abstandshalter (39) zwischen jedem magnetischen Material (32) umfasst, wobei der Abstandshalter (39) ein flexibles Material umfasst, welches eine Härte von bis zu ungefähr 55D aufweist,
wobei die magnetischen Materialien (32) derart sind, dass sie ein Magnetfeld zwischen einem äußeren Magneten (40) und den magnetischen Materialien (32) erzeugen, welches stark genug ist, dass es dem äußeren Magneten gestattet, den Führungsstab (30) innerhalb eines Patientenkörpers zu führen, wenn sich der äußere Magnet (40) ungefähr 100 mm (4 Zoll) von den magnetischen Materialien (32) entfernt befindet,
wobei das distale Ende (34) mit einer distalen Spitze (35) endet, und
wobei der Katheter (30) mit Magnetresonanztomographie (MRT) kompatibel ist.

2. Magensondenvorrichtung nach Anspruch 1, wobei der Führungsstab (30) ferner eine Reedschalteranordnung (60) umfasst.

3. Magensondenvorrichtung nach Anspruch 1, wobei das magnetische Material (32) aus der aus Magneten und magnetisch anziehenden Materialien bestehenden Gruppe ausgewählt ist.

4. Magensondenvorrichtung nach einem der Ansprüche 1 bis 3, wobei der Führungsstab (30) ferner eine von den magnetischen Materialien (32) distale Federdrahtführung (49) umfasst, wobei die Federdrahtführung (49) an der distalen Spitze (35) des Führungsstabs (30) endet.

5. Magensondenvorrichtung nach einem der Ansprüche 1 bis 4, ferner umfassend einen Signalgenerator (50), welcher mit dem proximalen Ende (31) des Führungsstabs (30) verbunden ist, wobei der Signalgenerator (50) einen Indikator (52) umfasst, welcher dazu in der Lage ist, ein Signal zu emittieren, welches aus der aus Licht, Schall, Vibration und digitaler Auslese bestehenden Gruppe ausgewählt ist.

6. Magensondenvorrichtung nach Anspruch 5, wobei der Indikator (52) eine lichtemittierende Diode (LED) ist.

7. Magensondenvorrichtung nach einem der vorhergehenden Ansprüche, wobei der Führungsstab (30) aus einem Dual-Durometer-Material gebildet ist.

8. Magensondenvorrichtung nach einem der vorhergehenden Ansprüche, wobei der Führungsstab (30) einen pH-Sensor an der distalen Spitze (35) des Führungsstabs (30) umfasst.

9. Magensondenvorrichtung nach einem der vorhergehenden Ansprüche, wobei die Wand (10) des Katheters (20) ein MRT kompatibles Verstärkungsmaterial umfasst.

10. Magensondenvorrichtung nach einem der vorhergehenden Ansprüche, wobei die Wand (10) des Katheters (20) eine glatte Innenlage (74), eine Binderlage (76), ein Verstärkungsmaterial (72) und eine Außenlage (78) umfasst.

11. Vorrichtung nach einem der vorhergehenden Ansprüche, wobei der Katheter (20) aus einem aus der aus Polyurethan- und Silikonschläuchen bestehenden Gruppe ausgewählt ist.

12. Vorrichtung nach einem der vorhergehenden Ansprüche, wobei die magnetischen Materialien (32) derart sind, dass sie ein Magnetfeld zwischen einem äußeren Magneten, welcher ein magnetisches Flussfeld von ungefähr 0,03 Tesla (300 Gauss) in einem Abstand von 100 mm (4 Zoll) von dem äußeren Magneten (40) und den magnetischen Materialien (32) aufweist, und wobei das Magnetfeld stark genug ist, um dem äußeren Magneten (40) zu gestatten, den Führungsstab (30) innerhalb eines Patientenkörpers zu führen, wenn sich der äußere Magnet (40) ungefähr 100 mm (4 Zoll) von den magnetischen Materialien (32) entfernt befindet.

## Revendications

1. Appareil de sonde d'alimentation (10) comprenant un cathéter (20) et un stylet amovible pouvant être guidé magnétiquement (30), dans lequel le stylet (30) comprend une extrémité proximale (31) formée dans un premier matériau ayant une valeur de module d'élasticité en flexion d'environ 862 MPa (125.000 psi) à environ 2758 MPa (400.000 psi) et une extrémité distale (34) formée dans un deuxième matériau différent du premier matériau et ayant une valeur de module d'élasticité en flexion allant jusqu'à 862 MPa (125.000 psi), dans lequel l'extrémité distale (34) est plus flexible que l'extrémité proximale (31), dans lequel le stylet (30) peut être inséré dans le cathéter (20) et peut être retiré de celui-ci, dans lequel l'extrémité distale du stylet (30) comprend plus d'un matériau magnétique (32) et une pièce d'espacement (39) entre chaque matériau magnétique (32), dans lequel la pièce d'espacement (39) comprend un matériau flexible ayant une dureté allant jusqu'à environ 55D,
dans lequel les matériaux magnétiques (32) sont aptes à créer un champ magnétique entre un aimant extérieur (40) et les matériaux magnétiques (32) qui est suffisamment puissant pour permettre à l'aimant externe de guider le stylet (30) à l'intérieur du corps d'un patient lorsque les aimants externes (40) sont à environ 100 mm (4 pouces) des matériaux magnétiques (32),
dans lequel l'extrémité distale (34) se termine par une pointe distale (35) et
dans lequel le cathéter (20) est compatible avec l'imagerie par résonance magnétique (IRM).

2. Appareil de sonde d'alimentation suivant la revendication 1, dans lequel le stylet (30) comprend en outre un commutateur à lames (60).

3. Appareil de sonde d'alimentation suivant la revendication 1, dans lequel le matériau magnétique (32) est sélectionné parmi le groupe constitué d'aimants et de matériaux attirés magnétiquement.

4. Appareil de sonde d'alimentation suivant l'une des revendications 1 à 3, dans lequel le stylet (30) comprend en outre un fil ressort guide (48) distal par rapport au matériau magnétique (32), dans lequel le fil ressort guide (48) se termine à la pointe distale (35) du stylet (30).

5. Appareil de sonde d'alimentation suivant l'une des revendications 1 à 4, comprenant en outre un générateur de signaux (50) connecté à l'extrémité proximale (31) du stylet (30), dans lequel le générateur de signaux (50) comprend un indicateur (52) capable d'émettre un signal sélectionné parmi le groupe comprenant un signal lumineux, un signal sonore, une vibration et un affichage numérique.

6. Appareil de sonde d'alimentation suivant la revendication 5, dans lequel l'indicateur (52) est une diode électroluminescente (LED).

7. Appareil de sonde d'alimentation suivant l'une des revendications précédentes, dans lequel le stylet (30) est formé dans un matériau composite de coextrusion.

8. Appareil de sonde d'alimentation suivant l'une des revendications précédentes, dans lequel le stylet (30) comprend une sonde de pH à l'extrémité distale (35) du stylet (30).

9. Appareil de sonde d'alimentation suivant l'une des revendications précédentes, dans lequel la paroi (70) du cathéter (20) comprend un matériau de renforcement compatible avec l'IRM.

10. Appareil de sonde d'alimentation suivant l'une des revendications précédentes, dans lequel la paroi (70) du cathéter (20) comprend une couche intérieure douce (74), une couche de liant (76), un matériau de renforcement (72) et une couche extérieure (78).

11. Appareil suivant l'une des revendications précédentes, dans lequel le cathéter (20) est formé dans un matériau sélectionné parmi le groupe comprenant le polyuréthanne et du tube en silicone.

12. Appareil suivant l'une des revendications précédentes, dans lequel les matériaux magnétiques (32) sont appropriés pour créer un champ magnétique entre un aimant externe qui a un champ de flux magnétique d'environ 0,03 tesla (300 gauss) à une distance de 100 mm (4 pouces) de l'aimant externe (40) et les matériaux magnétiques (32), et dans lequel le champ magnétique est suffisamment puissant pour permettre à l'aimant externe (40) de guider le stylet (30) à l'intérieur du corps d'un patient lorsque l'aimant externe (40) est à environ 100 mm (4 pouces) des matériaux magnétiques (32).
